# EUROPEAN PATENT APPLICATION

(11) **EP 4 652 992 A1**
(43) Date of publication of application: **26.11.2025**
(21) Application number: 25177926.0
(22) Date of filing: 21.05.2025
(51) Int. Cl.: A61K 31/015, A61K 31/382, A61K 31/498, A61K 9/00, A61K 45/06, A61P 27/02, A61P 27/04

(54) **OPHTHALMIC COMPOSITION FOR PREVENTING AND TREATING CONJUNCTIVA EPITHELIUM DAMAGE AND ASSOCIATED DISCOMFORT CAUSED BY HYPOTONIZING FORMULATIONS**

(30) Priority: 23.05.2024 IT 202400011671
(71) Applicant: FB Vision S.p.A., 63074 San Benedetto del Tronto (AP) (IT)
(72) Inventor: LATEGANO, Paolo, 63074 SAN BENEDETTO DEL TRONTO AP (IT); CARBONI, Paolo, 63074 SAN BENEDETTO DEL TRONTO AP (IT); GITTI, Gian Luca, 00127 ROMA (IT); IPAVEC, Valeria, 00148 ROMA (IT); GIFUNI, Consiglia, 20149 MILANO (IT); IORIO, Roberto, 67100 L'AQUILA (IT); CELENZA, Giuseppe, 67045 LUCOLI AQ (IT); PETRICCA, Sabrina, 67039 SULMONA AQ (IT); MAGAGNINI, Mattia, 60022 CASTELFIDARDO AN (IT)
(74) Representative: Modiano, Micaela Nadia

(57) **Abstract**

An ophthalmic composition comprising beta-caryophyllene and preferably hyaluronic acid for treating epithelial damage to the sclera or cornea of the eye caused by hypotonizing ophthalmic therapy, and reducing the associated sensation of discomfort and local side effects.

## Description

The present invention relates to an ophthalmic composition for preventing and treating conjunctiva epithelium damage caused by the instillation of ophthalmic preparations that reduce intraocular pressure and the associated pain and discomfort.

The invention originates in the ophthalmic sector, specifically in the sector of ophthalmic preparations for external use.

In particular, the present invention relates to an ophthalmic composition for eye instillation, which is adapted to preserve the cells of the ocular surface and of the conjunctiva epithelium from the damage caused by the instillation of medicines to reduce intraocular pressure and the associated sensation of pain or discomfort.

Glaucoma is a diverse group of eye diseases, all of which feature the presence of a chronic and progressive injury to the optic nerve, with characteristic alterations of the aspect of the optic nerve "head" and of the layer of retinal nerve fibers. Specifically, it is a neuro-degenerative disease characterized by the death of the ganglion cells of the retina.

Typically, glaucoma is diagnosed by measuring intraocular pressure and by examining the ocular fundus.

Glaucoma is a disease that is mostly asymptomatic and has a chronic profile; the patient reports a progressive loss of field of view. If not diagnosed early, glaucoma can cause irreversible damage, sight defects and, in the most serious cases, visual impairment or blindness. In the more advanced stages of the disease, patients report "tunnel vision", caused by the loss of peripheral vision and by vision limited to the center of the field of view.

The mechanisms by which glaucoma develops are still partially unknown. However, some general risk factors are known, such as patient age, family history, and also some more specific factors, such as high intraocular pressure and increased central corneal thickness.

Primary open-angle glaucoma is the most common form of this disease and represents one of the leading causes of blindness in the industrialized world. In this form of the disease, even though the drainage angle is "open", the fluid drains too slowly through the trabecular meshwork.

Patients who report the characteristic alterations of the optic nerve and of the corresponding field of view are treated independently of measurement of intraocular pressure. However, lowering the intraocular pressure remains the only clinically-proven treatment.

Currently, for glaucoma there are three different approaches to treatment: the first is treatment with medicines, the second laser surgery, and the third incisional surgery. The type of treatment to be used is determined by the type and seriousness of the disease.

Generally, treatment with medicines and most laser trabeculoplasty operations modify the secretion of aqueous humor and drainage paths.

Surgery is used only in the most serious cases of glaucoma. Traditional incisional surgery techniques, for example guarded filtration procedures, implantation of drainage devices, or valve shunts, are aimed at creating a new drainage route between the front chamber and the subconjunctival space. Other types of incisional surgery improve trabecular or uveoscleral outflow without creating a full-thickness fistula.

The initial forms of ocular hypertension and glaucoma are treated with ophthalmic preparations for local administration, aimed at reducing intraocular pressure and delaying or limiting the onset of more serious forms of glaucoma.

Typically, ophthalmic formulations are used, based on hypotonizing analogs of prostaglandin or beta-blockers, or oral or parenteral treatment is prescribed with carbonic anhydrase inhibitors. Suitable analogs of prostaglandin include bimatoprost, latanoprost, tafluprost, and travoprost. These active ingredients increase uveoscleral outflow, instead of altering the traditional system of aqueous humor outflow (trabecular/canalicular). Suitable beta-blockers include timolol, betaxolol, carteolol, levobunolol, and metipranol. These active ingredients reduce the production of aqueous humor without affecting the size of the pupil.

An additional active ingredient that is widely used as a topical hypotonizing compound is bromidine, an alpha-adrenergic receptor agonist.

Suitable carbonic anhydrase inhibitors include acetazolamide and matazolamide. These medicines are administered orally or parenterally, and reduce the production of aqueous humor. As glaucoma progresses, generally a combined treatment of these active ingredients is used.

Typically, hypotonizing active ingredients for topical use are formulated as single-dose ophthalmic preparations or as primary packs, typically in the form of eye drops or gels, free from preservatives like benzalkonium chloride.

However, topical use of these ophthalmic formulations can lead to side effects on the conjunctiva and the cornea, which are intrinsically connected with the type of medicine instilled. The onset of these secondary effects and their worsening as a consequence of long-term use of hypotonizing preparations contribute to the patient's poor compliance with the treatment, with the consequent increased risk of a rise in intraocular pressure and of the worsening of glaucomatous damage and loss of field of view.

It has also been observed that an integral ocular surface, not affected by OSD (Ocular Surface Disease), represents a critical factor for the success of filtration surgery for treating glaucoma, which, in a certain percentage of patients, can follow pharmacological treatment.

At present the need is felt therefore to have a product for ophthalmic use that reduces the onset of the local side effects that are intrinsically associated with the eye instillation of medicines with hypotonizing activity.

Therefore a general aim of the present invention is to provide an ophthalmic composition or preparation that is adapted to reduce the toxic effects on the ocular surface and related symptoms caused by topical hypotonizing therapy.

A further object consists in providing an ophthalmic composition for local use that increases the compliance of patients affected by intraocular hypertension and/or glaucoma with hypotonizing ophthalmic therapy, and/or that reduces the number of cases of interrupting the treatment or of irregular compliance.

A further object consists in providing an ophthalmic preparation that reduces the local discomfort or pain that accompanies the instillation of anti-glaucoma eye drops on the external anatomical structures of the eye.

The present invention originates from the discovery that the administration of an ophthalmic composition containing beta-caryophyllene, preferably in combination with hyaluronic acid, optionally in salified or cross-linked form, substantially reduces the discomfort and local symptoms that accompany the eye instillation of the ophthalmic hypotonizing formulations commonly used to reduce intraocular pressure, such as for example the ophthalmic preparations for treating open-angle glaucoma.

Advantageously the topical administration of an ophthalmic composition containing beta-caryophyllene, preferably in combination with hyaluronic acid, optionally salified or cross-linked, preserves the cells of the ocular surface from the damage induced by the active ingredients used to reduce intraocular pressure. This protection could also favor the greater success of a possible filtration surgery, in the advanced stages of the disease.

According to a general aspect, the present invention therefore provides an ophthalmic composition that comprises β-caryophyllene and preferably hyaluronic acid, optionally in salt form or in cross-linked form, for simultaneous, separate or sequential topical use with an ophthalmic preparation containing a hypotonizing active ingredient to treat epithelial damage for example to the sclera or cornea of an eye and reduce discomfort or local side effects caused by hypotonizing ophthalmic therapy.

According to another aspect, an ophthalmic composition is provided that comprises beta-caryophyllene and preferably hyaluronic acid, optionally in salt form or in cross-linked form, for simultaneous, separate or sequential topical use with a hypotonizing local ophthalmic preparation or treatment to improve the subject's compliance with treatment with the hypotonizing medicine.

According to some embodiments of the invention, the ophthalmic composition for the uses described here is suitable for simultaneous, separate, or sequential administration with an ophthalmic preparation containing a hypotonizing active ingredient. According to some embodiments, in the case of separate or sequential administration with a hypotonizing medicine, said administration occurs one hour before or after the administration of the hypotonizing ophthalmic preparation.

The high safety and tolerance profile of beta-caryophyllene makes the composition for the uses described herein particularly suitable in the treatment and/or in the prevention of irritating, painful and inflammatory phenomena that accompany the topical ophthalmic use of a hypotonizing ophthalmic medicine, typically in the form of eye drops or gel.

The ophthalmic composition described herein is used in the treatment of the principal side effects encountered with local hypotonizing treatment, which include stinging, in particular at the moment of instillation, a sensation of discomfort and/or of a foreign body, conjunctival hyperemia, and reddened eye.

According to some embodiments, the ophthalmic composition described herein comprises a physiologically acceptable vehicle and is in a pharmaceutical form that is suitable for local administration on the surface of the eye and adjoining tissues.

For example, the ophthalmic composition can be in liquid or gel form, advantageously provided with chemical/physical characteristics, for example pH and/or osmolarity, that mimic those of natural tears.

Preferably the physiologically acceptable vehicle is water-based, typically sterile. The vehicle of the ophthalmic composition described herein can furthermore contain components in liquid form that are suitable for ophthalmic administration.

According to some embodiments, the ophthalmic composition described herein is in liquid form, based on water or gel, or on a nano- and/or micro-micellar or optionally liposomal formulation, and is adapted to be instilled or applied on the outer surface of the eye, typically on the bulbar tunica externa, conjunctiva and/or on the rim of the eyelid.

For the purposes of the present invention, the active ingredient in preventing or treating the discomfort and/or pain associated with instillation in the eye of a hypotonizing ophthalmic and/or anti-glaucomatous product comprises β-caryophyllene, preferably in combination with hyaluronic acid, preferably in salt form. According to some embodiments, the hyaluronic acid is cross-linked with a physiologically acceptable cross-linking agent.

According to some preferred embodiments, the ophthalmic composition comprises β-caryophyllene in combination with hyaluronic acid in free form, optionally in salified form with a physiologically acceptable salt, or in cross-linked form.

Advantageously, the water-based ophthalmic composition described herein is formulated as eye drops or gel, adapted to be instilled on the surface or outer layer of the eye of a patient needing treatment. For example, the ophthalmic composition can be applied locally, directly on the sclera or cornea of an eye of a mammal.

The eye drops can be packaged as single-dose, of the disposable type with no preservatives, in order to reduce the risk of inflammation of the eye.

According to some embodiments, the ophthalmic composition described herein does not contain preservatives in order to reduce the risks of inflammation of the surface structures of the eye, or of causing or exacerbating the discomfort caused by local treatment with a hypotonizing medicine.

Preferably, the ophthalmic composition for the uses described herein does not contain disinfectants such as benzalkonium chloride, in order to reduce the incidence of irritant or inflammatory phenomena.

The local administration of the ophthalmic composition described herein reduces the nuisance and/or side effects that typically accompany the local administration of a hypotonizing ophthalmic and/or anti-glaucomatous preparation, so aiding compliance with the program of hypotonizing/anti-glaucomatous treatment by the patient needing the treatment.

In particular, it has been surprisingly observed that the ophthalmic administration of the ophthalmic composition according to any of the embodiments described herein has a soothing action on the outer anatomical structures of the eye, reducing the symptoms that typically accompany the instillation of hypotonizing eye drops, such as irritation, discomfort, and pain.

Advantageously, this soothing action on the surface structures of the eye is particularly pronounced when the ophthalmic composition described herein contains or is formulated with a phospholipid.

Consequently, according to some preferred embodiments, the composition described herein contains a physiologically acceptable vehicle containing phospholipids.

According to some embodiments, the ophthalmic composition described herein can contain one or more further substances adapted for the formulation of an ophthalmic composition such as, for example, a buffer or buffer system and one or more isotonic agents used in ophthalmic formulations.

According to some embodiments, the ophthalmic composition described herein can have a pH from slightly basic to neutral, for example, a pH comprised from 4 to 8.5, preferably from 6 to 7.8, more preferably from 6.8 to 7.4.

Advantageously the ophthalmic compositions described herein are ophthalmic compositions for medical use.

The ophthalmic composition for the uses described herein has a local soothing action with a wide margin of safety.

Some aspects and effects of embodiments of the ophthalmic composition described herein will become more evident from the accompanying figures, which are briefly described below.

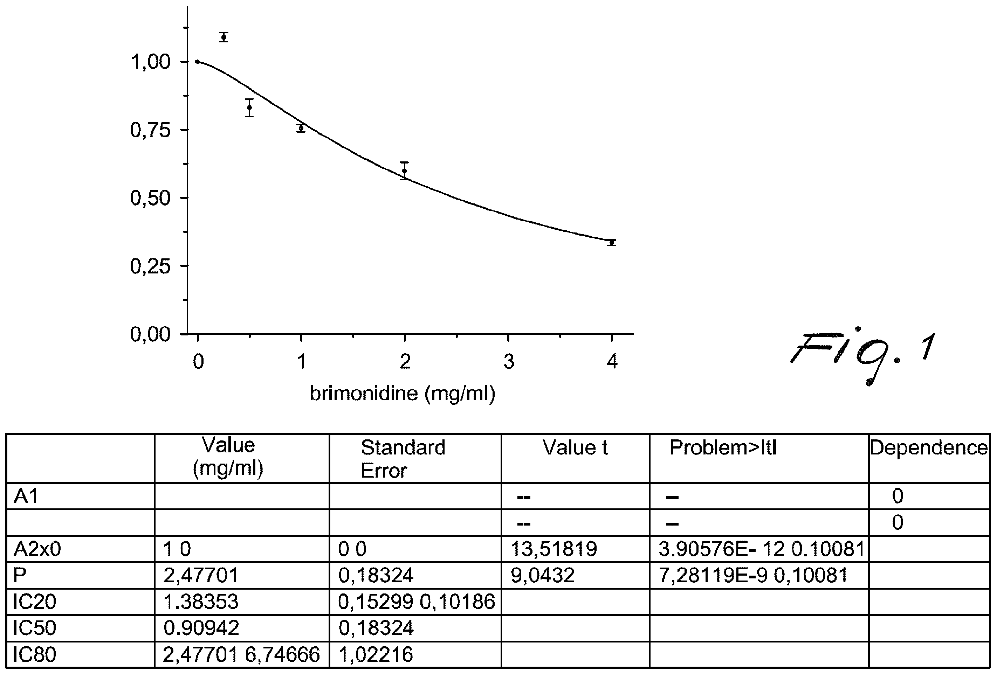

Figure 1 graphs the cytoprotective activity of beta-caryophyllene (BCP) on HCE cells exposed to concentrations increasing from 0.25 up to 4 mg/ml of hypotonizing brimonidine tartrate (BRIM), as described in Example 2. The underlying table shows the *IC50* values at 48 hours for BRIM. The data represent the average of three independent experiments ±
SE.

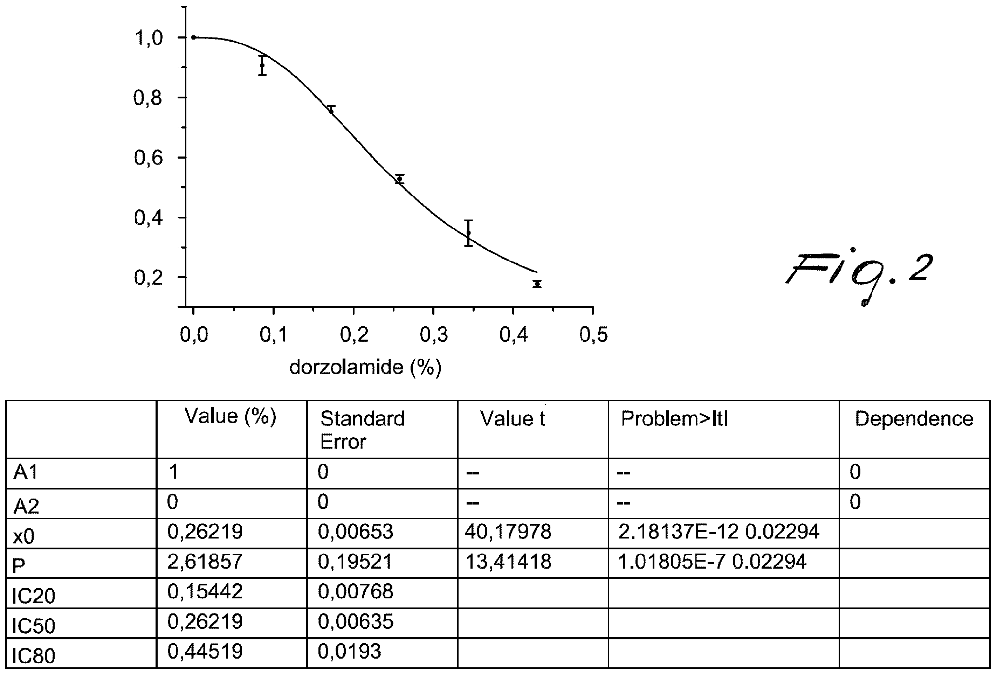

Figure 2 graphs the cytoprotective activity of beta-caryophyllene (BCP) on HCE cells exposed to concentrations increasing from 0.086 up to 0.43% mg/ml of hypotonizing dorzolamide (DZL). The underlying table shows the *IC50* values at 48 hours for DZL. The data represent the average of three independent experiments ± SE.

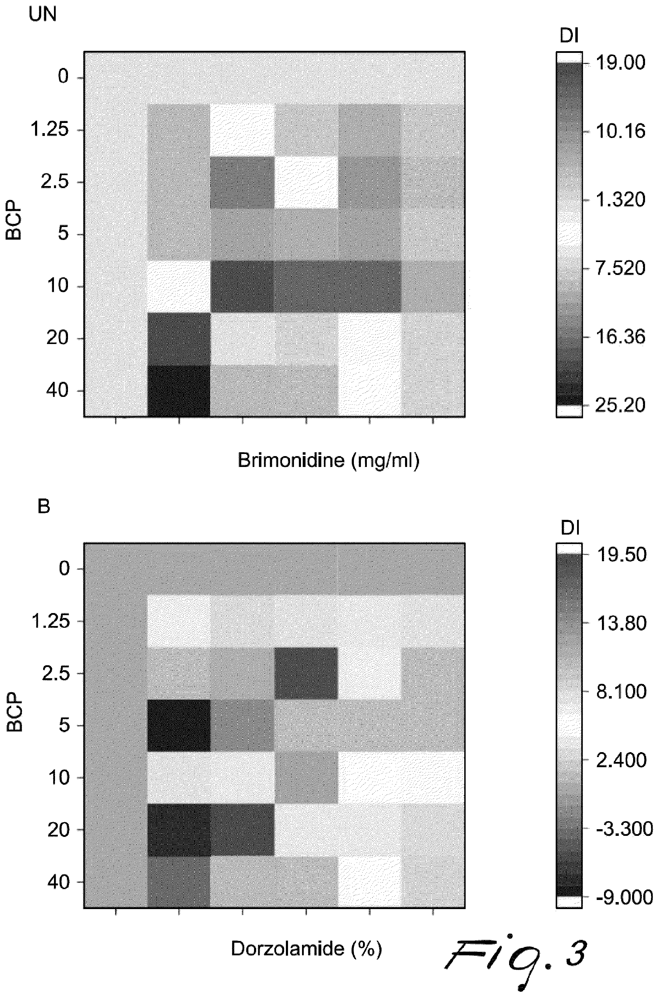

Figure 3 illustrates the *in vitro* interaction between BCP and BRIM (A) or DZL (B) determined by the ÿE model of non-parametric response surface analysis, as described in Example 2.

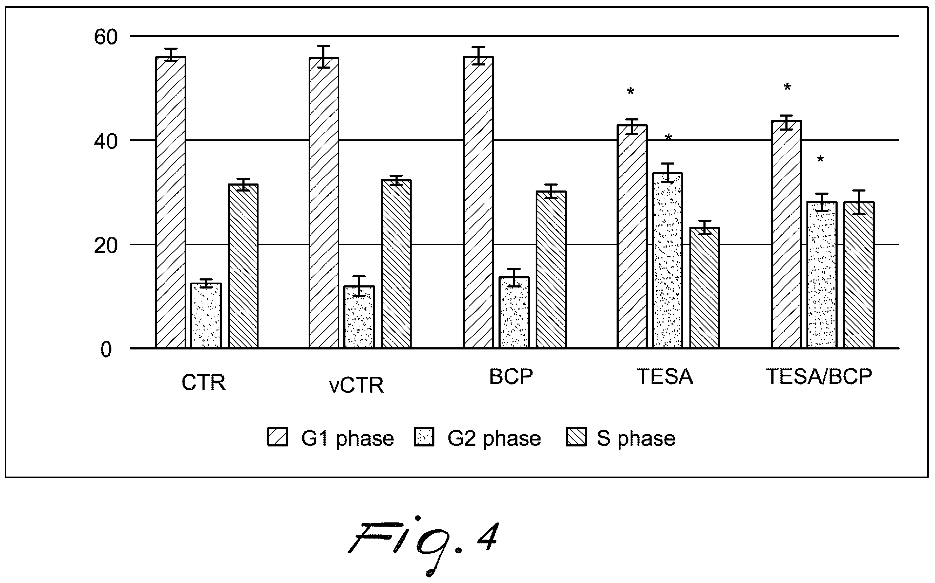

Figure 4 is a bar graph showing the effects of BRIM and BCP, on their own or in combination (48 hours of incubation), on cell cycle distribution in HCE cells. The distribution of cell cycle phases is shown using PI dyeing and analyzed using flow cytometry. The bar charts show the percentage of cells in each phase and represent the average ± SE of the results of three independent experiments; one-way ANOVA followed by Bonferroni's t-test; *p<0.05 with respect to vCTR.

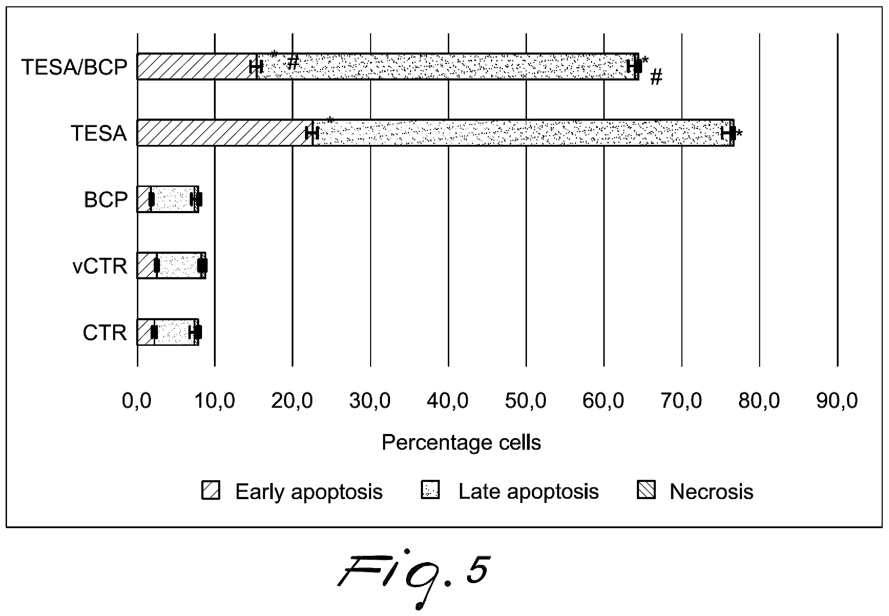

Figure 5 is a bar graph showing the percentage values of apoptotic cells (early and late) and necrotic cells in experimental groups exposed for 48 hours to BRIM and BCP. The data for three independent experiments, as illustrated in Example 2, are given as averages ± SE; one-way ANOVA followed by the Holm-Šidák method; *p<0.05 with respect to vCTR; #p<0.05 BRIM/BCP with respect to BRIM.

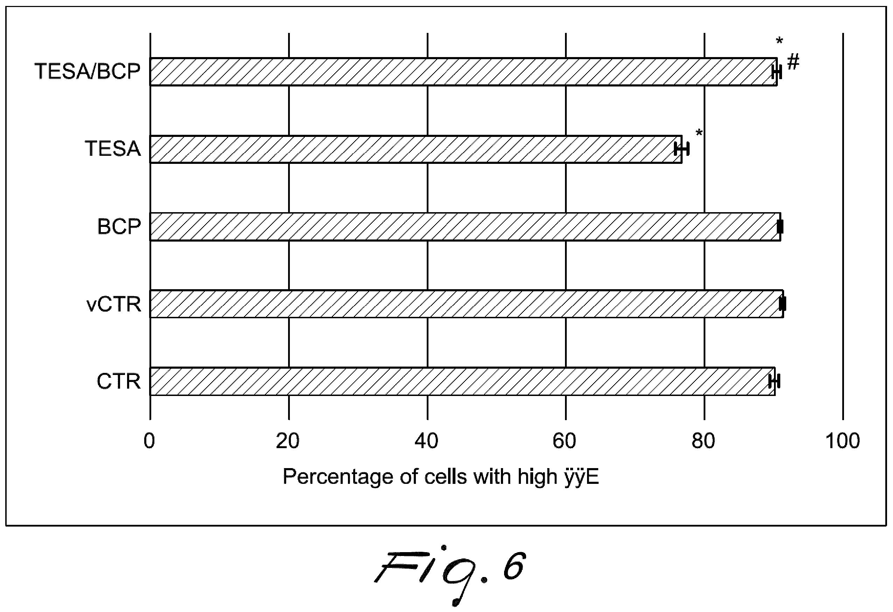

Figure 6 is a bar graph showing the effects of BRIM and BCP, on their own or in combination, on mitochondrial membrane potential (ÿÿm) in HCE cells after 24 hours of incubation. After the treatments, the ÿÿm was analyzed using JC-1 dye. The values represent the average ± SE of the results of three independent experiments; one-way ANOVA followed by the Holm-Šidák method; *p<0.05 with respect to vCTR; p<0.05 BRIM/BCP with respect to BRIM.

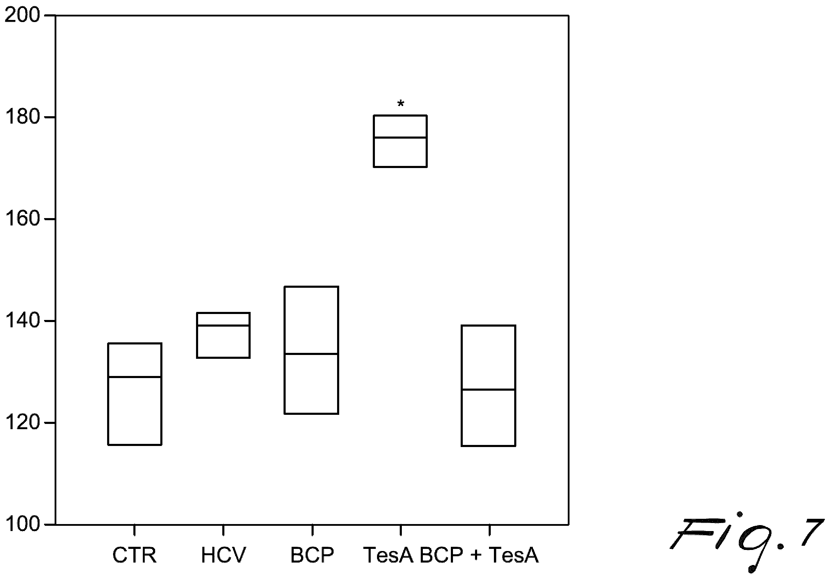

Figure 7 shows squares illustrating the effects of BRIM and BCP, on their own or in combination, on intracellular ROS generation in HCE cells. After 24 hours of treatment, intracellular production of ROS was analyzed using DCHF-DA with respect to vCTR. The values shown represent the average ± SE of the results of three independent experiments; one-way ANOVA followed by the Holm-Šidák method; *p<0.05 with respect to vCTR; p<0.05 BRIM/BCP with respect to BRIM dyeing with fluorescent dye. The values express the intensity of the fluorescence (Arbitrary Units, AU) and represent the average ± SE of the results of three independent experiments; one-way ANOVA in the rows; *p<0.05 with respect to vCTR.

According to some aspects of the invention, the local administration of an ophthalmic composition containing β-caryophyllene, preferably in combination with hyaluronic acid, reduces the sensation of discomfort or pain and the symptoms that accompany hypotonizing ophthalmic treatments.

An ophthalmic composition is described herein which comprises beta-caryophyllene, preferably in combination with hyaluronic acid, for treating epithelial damage to the sclera or cornea of the eye caused by hypotonizing ophthalmic therapy, and for reducing the associated sensation of discomfort and local side effects.

According to a general aspect, the invention described herein relates to an ophthalmic composition comprising β-caryophyllene as an active ingredient for simultaneous, separate or sequential topical use with a hypotonizing ophthalmic and/or anti-glaucomatous preparation to reduce the sensation of discomfort and local side effects ascribable to long-term instillation with hypotonizing medicines.

In the present discussion, the term "hypotonizing" means any active ingredient with a local hypotonizing or anti-glaucomatous activity, or any medicine that contains it.

According to another aspect, it has been observed that the local soothing effect obtained via the eye instillation of an ophthalmic composition containing β-caryophyllene is increased when the composition also contains hyaluronic acid, optionally in the form of a physiologically acceptable salt or cross-linked with a physiologically acceptable cross-linking agent.

Therefore an object of the present invention is an ophthalmic composition for the use as defined in claim 1.

Preferably the ophthalmic composition for the uses described herein is in liquid or gel form.

According to certain aspects of the invention, the ophthalmic composition described herein has a specific application in the treatment or prevention of discomfort, irritation or pain in the eyes and/or the oxidative and mitochondrial stress of human conjunctival epithelial cells treated with hypotonizing medicines.

Further embodiments of the medical uses of the ophthalmic composition described herein are defined in the accompanying dependent claims 2-10.

According to some embodiments, the ophthalmic composition has a specific application in the prevention and/or treatment of irritation or pain in the eyes and the local symptoms associated with treatment with hypotonizing/anti-glaucoma medicines such as for example hypotonizing analogs of prostaglandin, for example bimatoprost, latanoprost, tafluprost, or travoprost, or beta-blockers, for example timolol, betaxolol, carteolol, levobunolol, or metipranol, or alpha-adrenergic receptor agonists, for example bromidine, or carbonic anhydrase inhibitors, for example acetazolamide or matazolamide, and mixtures of said hypotonizing medicines.

The ophthalmic composition described herein contains β-caryophyllene, also known by the acronym BCP. This active ingredient is a bicyclic sesquiterpene of natural origin, named under IUPAC nomenclature as (1*R*,4*E*,9*S*)-4,11,11-Trimethyl-8-methylidenebicyclo[7.2.0]undec-4-ene.

Typically this molecule contains a cyclobutane ring bonded with a 9-member ring with a *trans*-double bond and having the following structural formula:

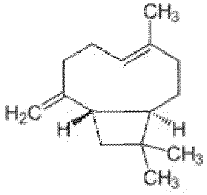

A suitable β-caryophyllene can be of synthetic or natural origin, for example in the form of a liquid extract, or for example a reconstituted or non-reconstituted dry extract, or an essential oil of plant origin, obtained from a plant.

According to some embodiments, the ophthalmic composition described herein contains β-caryophyllene, for example synthesized, liquid extract, reconstituted or non-reconstituted dry extract, or essential oil obtained from plants, in a quantity comprised from 0.001 to 3% by weight, preferably from 0.05 to 1%, more preferably from 0.1 to 0.5% by weight.

Preferably the ophthalmic composition further contains hyaluronic acid, optionally in salified form or in cross-linked form.

Hyaluronic acid typically is a glycosaminoglycan containing repeated disaccharide units formed by glucuronic acid joined by glycosidic bond β1→4 and β1→3 with _N_-acetylglucosamine.

A suitable hyaluronic acid can be in free form, non-cross-linked, or in salified form with a physiologically acceptable salt, for example sodium hyaluronate or potassium hyaluronate. In the ophthalmic composition described herein, the hyaluronic acid is preferably in salified form with sodium hyaluronate.

A suitable hyaluronic acid or its salt can have a medium/high molecular weight.

For example, a suitable hyaluronic acid or its salt can have an average molecular weight greater than 300,000 Da, preferably from 1,000,000 to 65,000,000, from 1,500,000 to 3,500,000 Daltons. In certain embodiments the ophthalmic composition comprises hyaluronic acid with at least two different molecular weights.

According to certain embodiments, the ophthalmic composition described herein comprises hyaluronic acid optionally in salified form, linear or cross-linked, in a quantity from 0.001 to 3% by weight, preferably from 0.05 to 1%, more preferably from 0.1 to 0.6% by weight, for example 0.3%.

According to some embodiments, the ophthalmic composition comprises β-caryophyllene in a quantity preferably from 0.001 to 3% by weight and hyaluronic acid optionally in salified or cross-linked form in a quantity from 0.001 to 3% by weight, preferably β-caryophyllene in a quantity comprised from 0.05 to 1% by weight and hyaluronic acid optionally in salified or cross-linked form in a quantity comprised from 0.05 to 1% by weight, more preferably β-caryophyllene in a quantity comprised from 0.5 to 0.1% by weight and hyaluronic acid optionally in salified or cross-linked form in a quantity comprised from 0.5 to 0.1% by weight.

The hyaluronic acid can be cross-linked with a physiologically acceptable cross-linking agent.

Typically, the molecular weights of hyaluronic acid or its salts, as described herein, are given as average molecular weight (MW), expressed in Daltons. The average molecular weight of hyaluronic acid described herein can be determined using standard methods, commonly used in the pharmaceutical formulation sector, for example as described by Ueno et al., 1988, Chem Pharm Bull. 36, 4971-4975; Wyatt 1993, Anal Chim Acta 272: 1-40; Watt Technologies 1999 "Light Scattering University DAWN Course Manual" and "DAWN Eos Manual" Wyatt Technology Corp. Santa Barbara CA (USA).

Hyaluronic acid, which is rich in hydroxyl groups that attract and retain water molecules, thickens and stabilizes the lacrimal film. Furthermore, hyaluronic acid reduces the effects of mechanical trauma on the ocular surface by means of lubrication and aiding re-epithelialization. Furthermore, hyaluronic acid reduces the rate of evaporation of tears from the ocular surface, so preventing and reducing symptoms.

It has been observed that the instillation of a composition containing β-caryophyllene and preferably hyaluronic acid reduces pain and the sensation of discomfort generated by corneal stress, which accompanies a topical hypotonizing treatment.

According to some embodiments, the ophthalmic composition comprises β-caryophyllene and hyaluronic acid optionally in salt or cross-linked form and a physiologically acceptable vehicle in liquid form. This formulation is suitable for providing an ophthalmic composition in the form of eye drops which is applied with a dropper into the eye to increase compliance with a long-term regime of hypotonizing ophthalmic treatment. The eye drops can be single-dose or multi-dose.

According to certain embodiments, the composition for the use described herein contains beta-caryophyllene in a quantity from 0.001 to 3% by weight, preferably from 0.05 to 1%, more preferably from 0.1 to 0.5% by weight in combination with said hyaluronic acid in any quantity comprised from 0.001 to 3% by weight, preferably from 0.05 to 1%, more preferably from 0.1 to 0.5% by weight. Advantageously the composition for the use described herein can contain any combination of the quantities of beta-caryophyllene and hyaluronic acid described above.

According to certain embodiments, the ophthalmic composition described herein has a pH in the range from 7.6 to 6.8 and incorporates a suitable buffer, for example selected from sodium hydrogen phosphate, in particular monohydrate, disodium phosphate, in particular dodecahydrate, sodium citrate, boric/borate acid and mixtures thereof.

In certain embodiments, the ophthalmic composition can contain calcium and magnesium salts, for example magnesium chloride and calcium chloride.

According to some embodiments, the ophthalmic composition described herein can comprise an isotonic agent, for example selected from magnesium chloride, calcium chloride, sodium chloride and mixtures thereof.

According to some embodiments, the ophthalmic composition described herein can comprise an additional isotonic agent such as for example glycerol.

According to some aspects, the ophthalmic composition described herein can be in single-dose form or multi-dose form. Typically, multi-dose preparations are supplied in containers that enable the administration of drops of the ophthalmic composition/preparation in succession.

According to certain embodiments, the ophthalmic composition or the ophthalmic preparations described herein also contain the matrix of nano/micromicelles and liposomes such as soya lecithin with or without vitamin E, TPGS, and surfactants such as for example polysorbates or derivatives of polyoxyethylene.

Advantageously the ophthalmic composition described herein can be isotonic, with lacrimal liquid, and preferably have an osmolarity from 260 to 320 mOsm/L, more preferably comprised between 275 and 300 mOsm/L.

Preferably the composition of the invention is in the form of ophthalmic eye drops or gel. For the purposes of administration, the above compositions can be provided as eye drops or in liquid or gel form.

The compositions described herein, when they are in the form of multi-dose preparations, can also contain preservatives with antimicrobial activity. Typical examples include parabens, quaternary ammonium salts, NIG/EDTA, polyhexamethylene biguanide (PHMB) and mixtures thereof.

As used herein, the term "combination" means that the components or active ingredients of the composition described herein are both present without the formation of any kind of chemical bond or other chemical/physical interaction between them.

The following examples further show some aspects of the invention.

### EXAMPLE 1

| FLUID EXTRACT FROM PLANT TITRATED TO 80% in BCP | | 0.125 |
|---|---|---|
| Phosphate buffer composed of: | | |
| | Potassium dihydrogen phosphate | 0.9 |
| | Disodium hydrogen phosphate | 0.5 |
| | Hydrochloric acid | 0.1 |
| Tween 80 | | 0.03 |
| Sodium chloride | | 0.570 |
| Lipoid 75S (soya lecithin) | | 0.35 |
| Hyaluronic acid | | 0.20 |
| Vitamin E TPGS | | 0.05 |
| water | | 100%, sufficient quantity |

### EXAMPLE 2

| FLUID EXTRACT FROM PLANT TITRATED TO 80% in BCP | 0.125 |
|---|---|
| Phosphate buffer composed of: | |
| Potassium dihydrogen phosphate | 0.9 |
| Disodium hydrogen phosphate | 0.5 |
| Hydrochloric acid | 0.1 |
| Tween 80 | 0.03 |
| Sodium chloride | 0.570 |
| Lipoid 75S (soya lecithin) | 0.35 |
| Vitamin E TPGS | 0.05 |
| water | 100%, sufficient quantity |

### EXAMPLE 3

*In vitro* tests on human conjunctival epithelial cells (HCE) for the experimental verification of the cytoprotective and antioxidant activity of beta caryophyllene (BCP) on the oxidative damage induced by anti-glaucoma medicines (AD). Characterization of the cytoprotective effects of beta-caryophyllene (BCP) on the human conjunctival epithelial cells exposed to oxidative and mitochondrial stress. This stress mimics the irritant and inflammatory action of hypotonizing ophthalmic medicines on the external structures of the human eye.

### Background

Brimonidine tartrate (BRIM; alpha-agonist), dorzolamide (DZL; carbonic anhydrase inhibitor) and tafluprost (TFP; prostaglandin analog) represent the hypotonizing treatment used most commonly to treat glaucoma, for their high efficacy and convenience of use.

The long-term eye instillation of these medicines has toxic effects on the corneal/conjunctival surface, causing discomfort, irritation and inflammation at the ocular level. These effects lead to reduced tolerance of anti-glaucomatous eye drops, which is associated with reduced compliance on the part of the patient.

### Purpose

This study sets out to evaluate the potential cytoprotective and antioxidant activities of beta caryophyllene (BCP) against the oxidative damage induced by anti-glaucoma medicines (AD) in human conjunctival epithelial cells (HCE). These activities are associated with an analgesic/antiinflammatory effect and with a reduction in the sensation of irritation and/or discomfort in the eyes experienced by patients being treated with a hypotonizing medicine for local use.

### The cytoprotective activity of BCP against the anti-proliferative effects induced by BRIM and DZL.

The HCE cells were exposed for 24 and 48 hours with increasing concentrations of BRIM (from 0.25 up to 4 mg/mL), DZL (from 0.086 up to 0.43%) and TFP (from 0.9 up to 30 µg/mL), alone and in combination with various concentrations of BCP (from 1.25 up to 40 µM), and the cell growth and vitality were evaluated respectively using the trypan blue exclusion test and the MTT assay. Significant protective effects of BCP in the cells treated with BRIM and DZL were observed at 48 hours of incubation, while no cytotoxic effects of TFP were recorded up to concentrations of 30 µg/mL. The *IC50* (average inhibitory concentration) values for BRIM and DZL on their own are given respectively in the accompanying Figures 1 and 2. The *in vitro* interactions between BCP and BRIM or DZL, as illustrated in Figures 3A and B, were determined by the ÿE model of non-parametric response surface analysis. Therefore, analysis of the medicine-medicine interaction showed an overall combinatorial effect of BCP in mitigating the cytotoxicity induced by both BRIM and DZL. In particular, the most advantageous combinations were 10 µM BCP with 2 mg/ml BRIM and 2.5 µM BCP with 0.25% DZL. Of the combinations selected, only the combination 10 µM BCP and 2 mg/mL BRIM, which reflects the commercial formulation of the eye drops for treatment, was studied further. All the statistical comparisons were performed with respect to vCTR (vehicle control).

### BCP reduces the arrest of the cell cycle induced by BRIM in the G2/M phase and in the apoptosis.

The cells were treated for 24 hours and 48 hours, and cytofluorimetric analyses of the cell cycle phase were performed for the distribution (Figure 4) and the apoptosis (Figure 5).

The exposure to BRIM is significant; it increased the percentage of cells in the G2/M phase from 12% (vehicle control) to 33.73% after 48 hours of incubation and this effect was associated with significant decreases in the S phase.

At the same time, the presence/addition of BCP alleviated the arrest of the cell cycle in the G2/M phase (BRIM/BCP = 28.11%). Furthermore, the BCP also reduced the apoptosis induced by BRIM at 24 and 48 hours of incubation. Specifically, the early apoptosis was significantly reduced by approximately 35% at 24 and 48 hours, while the late apoptosis showed a decrease of approximately 15% at 48 hours.

### BCP restores the fall in the mitochondrial membrane potential (ÿÿm) induced by BRIM in a time-dependent manner.

The apoptosis process is intimately associated with mitochondrial dysfunction. Therefore, it was verified whether the cytotoxicity induced by BRIM was linked to the loss of mitochondrial membrane potential. Measurements of mitochondrial activity, performed by means of experiments over time (3 - 24 hours), were evaluated by means of coloring with JC-1 dye. At any analyzed moment, BRIM induced a marked mitochondrial depolarization, measured by a decrease in the percentage of cells with high mitochondrial membrane potential ÿÿm, as illustrated in the accompanying Figure 6 and associated description.

### BCP mitigates the imbalance induced by BRIM of the redox state in HCE cells.

To investigate whether the mitochondrial dysfunction induced by BRIM was associated with the accumulation of ROS, the HCE cells were exposed to BRIM, BCP and combinations thereof for 24 hours and then analyzed for DCFH-DA fluorescence. As shown in Figure 7, BRIM showed pro-oxidant properties. In particular, the medicine induced a significant increase in ROS production equal to approximately 30% (vs. vCTR); this effect, however, was not found in the combined BCP/BRIM treatment, so also proving an antioxidant capacity of BCP.

### CONCLUSIONS

In our experimental conditions *in vitro,* BCP effectively reduced the cytotoxicity induced by BRIM and DZL, promoting mitochondrial protection and antioxidant effects. Therefore, BCP represents a promising candidate for promoting eye health, by preventing or delaying the onset of eye diseases and disorders connected with hypotonizing treatment, in particular for improving tolerance of anti-glaucomatous eye drops associated with reduced patient compliance.

The disclosures in Italian Patent Application No. 102024000011671 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An ophthalmic composition comprising beta-caryophyllene, for simultaneous, separate or sequential topical use with a hypotonizing ophthalmic preparation to treat epithelial damage to the sclera or cornea of an eye and reduce discomfort or local side effects caused by hypotonizing ophthalmic therapy.

2. The composition for use according to claim 1, further comprising hyaluronic acid, optionally in salt form or in cross-linked form.

3. The composition for use according to claim 2, wherein the beta-caryophyllene is contained in an amount comprised from 0.001 to 3% by weight, preferably from 0.05 to 1%, more preferably from 0.1 to 0.5% by weight, and said hyaluronic acid is contained in an amount comprised from 0.001 to 3% by weight, preferably from 0.05 to 1%, more preferably from 0.1 to 0.5% by weight, in any combination of said amounts.

4. The composition for use according to claim 1 in the form of a sterile aqueous solution for eye instillation.

5. The composition for use according to claim 2, wherein the hyaluronic acid optionally in salt form has an average molecular weight greater than 300,000 Da, preferably comprised from 1,000,000 to 5,000,000, from 1,500,000 to 3,500,000 Daltons.

6. The composition for use according to any one of claims 1-5, wherein said hypotonizing ophthalmic preparation contains at least one prostaglandin analog, one beta-blocker, one alpha-adrenergic receptor agonist, one carbonic anhydrase inhibitor, and mixtures thereof.

7. The composition for use according to claim 6, wherein the prostaglandin analog is chosen from bimatoprost, latanoprost, tafluprost, travoprost and mixtures thereof, the beta-blocker is chosen from timolol, betaxolol, carteolol, levobunolol, metipranol and mixtures thereof, the alpha-adrenergic receptor agonist is bromidine, the carbonic anhydrase inhibitor is chosen from acetazolamide and/or matazolamide and mixtures thereof.

8. The composition for use according to any one of claims 1-7, furthermore comprising:
- a buffer or buffer system for adjusting the pH value, preferably chosen from sodium hydrogen phosphate, disodium phosphate, sodium citrate and mixtures thereof,
- an isotonic agent chosen from sodium chloride, potassium chloride, magnesium chloride and mixtures thereof.

9. The composition for use according to any one of claims 1-8 in the form of drops, eye drops or artificial tears for instillation into the eye, preferably free from preservatives and more preferably formulated as single-dose dosage units.

10. The ophthalmic composition comprising beta-caryophyllene and preferably hyaluronic acid optionally in salt or cross-linked form, for simultaneous, separate or sequential topical use with a hypotonizing ophthalmic medicine to improve the subject's compliance with treatment with the hypotonizing ophthalmic medicine.
